# EUROPEAN PATENT SPECIFICATION

(11) **EP 0 504 381 B1**
(45) Date of publication and mention of the grant of the patent: **14.06.1995**
(21) Application number: 91919339.1
(22) Date of filing: 03.10.1991
(51) Int. Cl.: C07C 227/02

(54) **METHOD FOR OBTAINING ACETIC ACID DERIVATIVES**
VERFAHREN ZUR HERSTELLUNG VON ESSIGSÄUREDERIVATEN
PROCEDE D'OBTENTION DE DERIVES DE L'ACIDE ACETIQUE

(30) Priority: 04.10.1990 ES 9002523
(43) Date of publication of application: 23.09.1992
(73) Proprietor: MONSANTO COMPANY, St. Louis, Missouri 63167 (US)
(72) Inventor: OCHOA GOMEZ, José, Ramón, E-28760 Tres Cantos (ES); MARTIN RAMON, Juan, Luis, E-28006 Madrid (ES)
(74) Representative: Bosch, Henry
(86) International application number: ES9100061
(87) International publication number: WO9206069

(56) References cited:
- GB-A- 2 148 287

## Description

The present invention relates to a method for obtaining derivatives of acetic acid, specifically glycine, iminodiacetic acid and nitrilotriacetic acid by oxidizing monoethanolamine, diethanolamine and triethanolamine respectively, using a Raney copper catalyst. In specific terms, the procedure of the invention refers to a regeneration treatment of the catalyst used in a previous reaction cycle which allows it to be used again in successive reactions, whilst maintaining a conversion and net yield in all trials carried out with the regenerated catalyst of the same order of magnitude as that obtained when using a new catalyst (85-92%).

### Background to the invention

Glycine, iminodiacetic acid (IDA) and nitrilotriacetic acid (NTA), described in numerous patents, have many applications in various fields. Glycine, for example, has applications as a nutrient and both IDA and NTA are intermediates in the synthesis of herbicides, among other applications. Since the appearance of these materials on the market, various methods for obtaining them have been described and patented.

The Japanese patent 53077009, in the name of Mitsui Toatsu Chemical Co. Ltd., provides a method for obtaining IDA and NTA by oxidizing diethanolamine and triethanolamine respectively, with gaseous oxygen in an alkaline aqueous solution employing a noble metal (Pt and Pd) as the catalyst of the reaction. Although this method enables the said acids to be obtained, the yield obtained for IDA (69%) as well as for NTA (60%) is rather low and it also has the disadvantage that possible losses of the noble metal employed as the catalyst make this process hardly economical. Moreover, it is known by experts in the catalytic field that the losses of noble metals that occur during catalytic processes, whether acidic or alkaline, through solution of the said metal in the reaction water, necessitate complex processes in order to recover the metal, in addition to those normal losses that occur during the handling of the said catalysts. Consequently, these types of processes are hardly profitable in the case of end products with limited added value.

It would therefore be advantageous to have a method that enabled adequately pure glycine, IDA and NTA to be obtained in good yields by oxidizing mono-, di- and triethanolamine respectively in the presence of a non-noble metal catalyst (for example, Raney copper), which would minimize the economic disadvantages indicated above.

In this regard, Japanese patents Nos. 60/78.948, 60/78.949, 60/97.945, 60/100.545 and 61/65.840, in the name of Nippon Catalytic Chem. Ind., provide a method for obtaining glycine, IDA and NTA by dehydrogenating mono-, di- and triethanolamine, respectively, in an alkaline aqueous solution employing Raney copper as the catalyst, but with oxygen obtained by the decomposition of water, according to the following equations:

C₂H₇ON + H₂O → C₂H₅O₂N + 2H₂ (glycine)

C₄H₁₁O₂N + 2H₂O → C₄H₇O₄N + 4H₂ (IDA)

C₆H₁₅O₃N + 3H₂O → C₆H₉O₆N + 6H₂ (NTA)

Although this method enables high yields of glycine, IDA and NTA to be obtained, the operation start-up causes loss of activity of the catalyst, which, together with the cost of the flameproof equipment required to carry out this method, directly affects the end production cost of the required product to the point where the process can become economically unviable.

A way of making this method economically viable would be to minimize the losses of the Raney copper catalyst. It would be particularly advantageous if the catalyst employed in the reaction cycle were simple and regenerated economically with the object of recovering its catalytic activity so that it could be reused in successive reaction cycles. None of the above-mentioned Japanese patents mentions the possibility of reusing or of regenerating the used Raney copper catalyst.

The present invention has therefore for its object to provide improvements to the method of dehydrogenating mono-, di- and triethanolamine in the presence of a Raney copper catalyst. The aim of these improvements is to regenerate the activity of the Raney copper catalyst through a simple regeneration method of the said catalyst which enables its reuse in successive reaction cycles, thereby rendering the said procedure of dehydrogenating mono-, di- and triethanolamine economically viable. Through the process of regenerating the catalyst, it recovers its catalytic activity and consequently may be introduced into other successive reaction cycles in order to obtain glycine, IDA or NTA with adequate yields and purity (of the same order of magnitude as that, obtained when a new catalyst is used, between 85% and 92%), which results in the production of these acids being economically profitable and industrially viable, which, would not be so in the case of glycine, IDA or NTA being produced using the catalyst immediately after its first use without prior regeneration or of its inability to be reused because its catalytic activity had become exhausted. Since the catalyst regenerated by the method of this invention can be reused on successive occasions, whilst maintaining the reaction conversions and yields, the cost effect of the catalyst in the end product is minimized.

### Brief description of the invention

The improvements provided by the invention may be summarized as minimizing the cost effect of the catalyst in the end products obtained, thereby rendering economically viable on an industrial scale a method which would otherwise not be viable. In order to minimize the effect of the catalyst on the end products, the invention proposes a method for regenerating the catalyst used in the reaction with formic acid under reflux, thereby allowing all the oxidized copper (Cu²⁺) which would be produced during the reaction process to be withdrawn from the used catalyst. Oxidized copper and other possible contaminating materials are precipitated onto the catalyst, poisoning it and producing considerably reduced yields of the reactions performed by reusing the catalyst without subjecting it to the regeneration process mentioned. These reduced yields are demonstrated in Examples 2 to 4 given in this description, in which it may be seen that the reaction yield after reusing the unregenerated catalyst drops to 36% in the fourth reaction cycle. If, on the contrary, the catalyst is regenerated, as will be described in detail below, when the catalyst regenerated according to the method of the invention is reused, the reaction yields are maintained at around 85-92% (Examples 5 to 9).

### Detailed description of the invention

The invention provides a series of considerable improvements compared to the patents referred to above in Background to the invention, as it minimizes the cost effect of the catalyst on the end products obtained. In these patents, a method is described for obtaining glycine, IDA and NTA using a Raney copper catalyst which may be represented by the following equations: 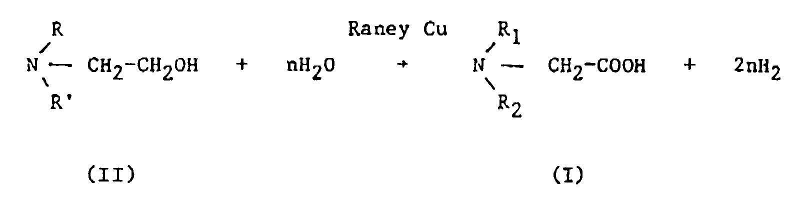
where R and R' may independently be H or groups -CH₂-CH₂OH;
R₁ and R₂ may independently be H or groups -CH₂-COOH;
n may be 1, 2 or 3.

As may be seen, when R = R' = H, the amino alcohol (II) is monoethanolamine and the compound (I) obtained is glycine (R₁ = R₂ = H). Similarly, when one of R or R' is H and the other is -CH₂-CH₂OH, the compound (II) is diethanolamine and the compound (I) obtained is iminodiacetic acid (one of R₁ or R₂ is H and the other is -CH₂-COOH), and when R = R' = -CH₂-CH₂OH the compound (II) is triethanolamine and the compound (I) obtained is nitrilotriacetic acid.

The reaction can be performed at temperatures between 25°C and 215°C, and preferably between 150°C and 205°C, and at a pressure between 6 and 15 kg/cm². Water is used as a solvent, to which an alkali metal hydroxide is added in a molar ratio compared to the initial amino alcohol hydroxide groups (II) of between 5% and 20% in stoichiometric excess, and preferably between 5% and 10%. This means that from 1.05 to 1.10 moles of alkali metal hydroxide per mole of monoethanolamine, between 2.10 and 2.20 moles of the said hydroxide per mole of diethanolamine and between 3.15 and 3.30 moles of alkali metal hydroxide per mole of triethanolamine are used. Any alkali metal hydroxide may be employed provided that the salts formed with the acids produced are soluble in the reaction medium. Examples of suitable alkali metal hydroxides are sodium hydroxide and potassium hydroxide.

The initial concentration of amino alcohols (II) may be between 20% and 35% by weight compared to the initial total weight of the components of the reaction mass, and preferably between 30% and 35% by weight. Higher concentrations may result in reduced yields due to problems of diffusion of oxidizing gas, and lower concentrations reduce productivity without improving the results.

The catalyst employed in the method of the invention is Raney copper, although it is also possible to use a Raney nickel catalyst if a few modifications are made to the process. The Raney copper catalyst is easily manufactured by leaching an Al₂Cu alloy with sodium hydroxide in a reducing atmosphere, according to procedures already described. The catalyst is added in a quantity between 5% and 15% by weight compared to the initial amino alcohol content (II), although with an average percentage of 7.5%, excellent results can be obtained.

The reaction time under these conditions is between 3.5 and 5.0 hours depending on the number of times the catalyst is used.

The reaction is continued by measuring the conversion of the initial amino alcohol (II) and the appearance of the ace tic acid derivative (I). Once the reaction is finished, the reaction medium undergoes a hot filtration process, at a temperature of between 80°C and 95°C, and preferably between 85°C and 90°C, with the aim of recovering the used catalyst. The regeneration method is then carried out on the used Raney copper catalyst according to the new process that is being claimed in the present patent application and which constitutes the essential claim of the patent. This regeneration method of the catalyst provides the improvements claimed and renders the above-mentioned method viable economically and on an industrial scale since, otherwise, the said method would not be viable for the reasons set out in the Background to the invention.

The Raney copper catalyst regeneration method of the present invention basically consists of treating the Raney copper catalyst that has already been used in a previous reaction cycle with formic acid under reflux so that subsequently, and after further filtration, it can be digested with demineralized water and sodium hydroxide diluted repeatedly until a slightly alkaline pH (9-11) in the wash water is achieved. The treated catalyst may be stored under a dilute aqueous solution of sodium hydroxide (0.5-1.0%) until it is employed in the next reaction cycle.

The concentration of formic acid to be employed may be between 20% and 85% by weight, and this concentration is preferably 40%.

The alkaline solution of the reaction medium, which contains the soluble alkali metal salts of the acetic acid derivatives formed, is isolated and treated to obtain the corresponding purified formula (I) acids. This treatment may be performed either chemically, by crystallization at temperatures between 75°C and 85°C, and preferably between 80°C and 85°C, of the reaction solution that has been previously treated with hydrochloric acid to obtain a pH between 0.5 and 2.5, depending on the acid obtained, or electrochemically, by an electrodialysis procedure as claimed in the Spanish patent application no. 9000130.

As may be seen from the examples given in this description, when the unregenerated Raney copper catalyst is reused, the reaction yield falls to 36% in the fourth reaction cycle, whilst reusing the catalyst regenerated by the procedure of the invention maintains the reaction yield in the fourth reaction cycle at around 87%, that is, of the same order or magnitude as that obtained when a new catalyst is employed (85-92%).

The present invention may be clearly illustrated by the following examples which should not be considered limitative of, but illustrative of the invention, and which enable the nature of its procedure to be more clearly understood.

### Example 1

In a flameproof, 2-litre capacity, stainless steel autoclave reactor (AISI 316), are loaded in the following order and with stirring: 263.7 g of 97% sodium hydroxide; 640 g of demineralized water; 326.4 g of 98% pure diethanolamine; 45.7 g of Raney copper with an approximate moisture content of 30%, which effectively means using 32 g net of catalyst in the trial and, finally, an additional 40 g of demineralized water to wash away any trace of the previous reagents. The inside of the reactor is then flushed twice with nitrogen (10 kg/cm²) and twice more with hydrogen (10 kg/cm²). The last sweep of hydrogen is depressurized to 2 kg/cm² and, with the reactor closed, heating of the reactants is commenced. Once 14-16 kg/cm² has been reached inside the reactor, it is depressurized to 10 kg/cm² and, with the vent open but maintaining the said internal pressure, heating is continued a 170-175°C, when the timing of the reaction begins. After four hours from the start of the reaction, it is considered finished and cooled to 90°C, filtered through a Buchner and Kitasato, and the Raney copper catalyst recovered. After washing well, it is stored under formic acid so that once it has been regenerated it may be reused in successive reactions. After analysis, the reaction solution provide a yield of 90.2% IDA in the form of its disodium salt which, when submitted to conventional electrodialysis, results in an almost quantitative recovery of free IDA with a purity above 98%.

### Examples 2 to 4

These experiments were carried out to establish the behaviour of the catalyst employed in Example 1, in three consecutive trials, without submitting the catalyst to any regeneration process. The three trials were performed using the same methodology as that used in Example 1. However, in these trials the catalysts employed always originated from the unregenerated one used in the previous trial, though allowing for estimated handling losses. The reaction yields obtained after 4 hours in Example 2 and after 4.5 hours in Examples 3 and 4 were 82.6%, 60.6% and 36% respectively.

### Examples 5 to 9

These experiments were carried out using the same methodology as that of Example 1. However, in these experiments the catalyst employed was new in the first trial and submitted to regeneration treatment in the four remaining trials. The catalyst regeneration in Examples 6 to 9, inclusive, was carried out as follows: 32 g net of catalyst already used in Example 5 were suspended in 13.6 g of 85% formic acid and 15.2 g of demineralized water. The suspension was placed in a 100 cc capacity glass flask and maintained under reflux for 1 hour, after which the catalyst was vacuum-filtered through a Buchner and Kitasato and digested initially with 32 g of demineralized water and after filtering again was digested with 24 g of 2% sodium hydroxide, thereby achieving a pH of approximately 10.32 in the final wash water. The treated catalyst was employed in the next trial. The yields obtained in the various experiments were 92.6%, 85.6%, 85.1%, 87.1% and 85.2%. The final average yield was 87.12%. In all cases, the free acids obtained were isolated and purified by eleotrodialysis.

The following table shows the yields obtained in Examples 2-4 and 6-9 (without regenerating and with regenerating the catalyst, respectively) in various reaction cycles. A significant reduction in the reaction yield from the third cycle can be seen when an unregenerated catalyst is employed.

| Reaction cycle | Yield % | |
|---|---|---|
| | U.C. | R.C. |
| 2 | 82.6 | 85.6 |
| 3 | 60.6 | 85.1 |
| 4 | 36 | 87.1 |
| 5 | - | 85.2 |
| U.C. Unregenerated catalyst. R.C. Regenerated catalyst according to the procedure of the invention. | | |

## Claims

1. A method for the preparation of derivatives of acetic acid such as glycine, iminodiacetic acid and nitrilotriacetic acid by dehydrogenating monoethanolamine, diethanolamine and triethanolamine, respectively, in the presence of a Raney copper catalyst, characterized in that it comprises submitting the catalyst used to a regeneration process by treating the said used catalyst with formic acid under reflux.

2. The method of claim 1, characterized in that the dehydrogenation reaction is carried out in the presence of an alkali metal hydroxide in an amount between 5 and 20 % in stoechiometic excess over that required to produce the salt of the said acetic acid derivative.

3. The mehtod of claim 1, characterized in that the catalyst treated with formic acid under reflux and filtered is digested with demineralized water and sodium hydroxide until the wash water reaches an alkaline pH between 9 and 11.

4. The method of any of claims 1 to 3, characterized in that the concentration of the formic acid employed is between 20 % and 85 % by weight.

5. The method of any of claims 1 to 4, characterized in that the reaction is carried out between 25°C and 215°C.

6. The method of claim 5, characterized in that the reaction is carried out between 150°C and 205°C.

7. A method for regenerating a Raney copper catalyst for use in the conversion of aminoalcohols selected from monoethanolamine, diethanolamine and triethanolamine to glycine, iminodiacetic acid and intrilotriacetic acid, respectively, characterized in that it comprises treating used Raney copper catalyst under reflux with formic acid.

8. The method of claim 7, characterized in that the Raney copper catalyst is further washed with demineralized water and sodium hydroxide until the wash water reaches a pH between 9 and 11.

9. The method of claim 7 or 8, characterized in that the concentration of formic acid is between 20 % and 85 % by weight.

## Patentansprüche

1. Verfahren zur Herstellung von Essigsäure-Derivaten, wie Glycin, Iminodiessigsäure und Nitrilotriessigsäure, durch das Dehydrieren von Monoethanolamin, Diethanolamin bzw. Trietanolamin, in Anwesenheit eines Raney-Kupferkatalisators, dadurch gekennzeichnet, daß es das Unterwerfen des verwendeten Katalysators einem Regenerationsverfahren durch die Behandlung des verwendeten Katalysators mit Ameisensäure am Rückfluß umfaßt.

2. Verfahren nach Anspruch 1, dadurch gekennzeichnet, daß die Dehydrierungsreaktion in Anwesenheit eines Alkalimetallhydroxids in einer Menge zwischen 5 und 20 % in einem stöchiometrischen Überschuß gegenüber jenem, der zur Herstellung des Salzes des Essigsäure-Derivats erforderlich ist, durchgeführt wird.

3. Verfahren nach Anspruch 1, dadurch gekennzeichnet, daß der mit Ameisensäure am Rückfluß behandelte und filtrierte Katalysator mit entmineralisiertem Wasser und Natriumhydroxid digeriert wird, bis das Waschwasser einen alkalischen pH zwischen 9 und 11 erreicht.

4. Verfahren nach einem der Ansprüche 1 bis 3, dadurch gekennzeichnet, daß die Konzentration der verwendeten Ameisensäure zwischen 20 Masse-% und 85 Masse-% beträgt.

5. Verfahren nach einem der Ansprüche 1 bis 4, dadurch gekennzeichnet, daß die Reaktion zwischen 25°C und 215°C durchgeführt wird.

6. Verfahren nach Anspruch 5, dadurch gekennzeichnet, daß die Reaktion zwischen 150°C und 205°C durchgeführt wird.

7. Verfahren zum Regenieren eines Raney-Kupferkatalysators zur Verwendung bei der Überführung von Aminoalkoholen, ausgewählt aus Monoethanolamin, Diethanolamin und Triethanolamin, in Glycin, Iminodiessigsäure bzw. Nitrilotriessigsäure, dadurch gekennzeichnet, daß es die Behandlung des verwendeten Raney-Kupferkatalysators am Rückfluß mit Ameisensäure umfaßt.

8. Verfahren nach Anspruch 7, dadurch gekennzeichnet, daß der Raney-Kupferkatalysator weiters mit entmineralisiertem Wasser und Natriumhydroxid gewaschen wird, bis das Waschwasser einen pH zwischen 9 und 11 erreicht.

9. Verfahren nach Anspruch 7 oder oder 8, dadurch gekennzeichnet, daß die Konzentration der Ameisensäure zwischen 20 Masse-% und 85 Masse-% liegt.

## Revendications

1. Procédé pour la préparation de dérivés de l'acide acétique tels que la glycine, l'acide iminodiacétique et l'acide nitrilotriacétique par déshydrogénation de la monoéthanolamine, de la diéthanolamine et de la triéthanolamine, respectivement, en présence d'un catalyseur de cuivre de Raney, caractérisé en ce qu'il comprend le fait de soumettre le catalyseur utilisé à un procédé de régénération par traitement dudit catalyseur utilisé avec de l'acide formique à reflux.

2. Procédé selon la revendication 1, caractérisé en ce que la réaction de déshydrogénation est effectuée en présence d'un hydroxyde de métal alcalin dans une quantité comprise entre 5 et 20 % en excès stoechiométrique par rapport à la quantité requise pour produire le sel dudit dérivé d'acide acétique.

3. Procédé selon la revendication 1, caractérisé en ce que le catalyseur traité avec l'acide formique à reflux et filtré est mis à digérer avec de l'eau déminéralisée et de l'hydroxyde de sodium jusqu'à ce que l'eau de lavage atteigne un pH alcalin compris entre 9 et 11.

4. Procédé selon l'une quelconque des revendications 1 à 3, caractérisé en ce que la concentration de l'acide formique employée est comprise entre 20 % et 85 % en poids.

5. Procédé selon l'une quelconque des revendications 1 à 4, caractérisé en ce que la réaction est effectuée entre 25°C et 215°C.

6. Procédé selon la revendication 5, caractérisé en ce que la réaction est effectuée entre 150°C et 205°C.

7. Procédé de régénération d'un catalyseur de cuivre de Raney pour utilisation dans la transformation d'aminoalcools choisis parmi la monoéthanolamine, la diéthanolamine et la triéthanolamine en glycine, acide iminodiacétique et acide nitrilotriacétique, respectivement, caractérisé en ce qu'il comprend le traitement du catalyseur de cuivre de Raney utilisé avec de l'acide formique à reflux.

8. Procédé selon la revendication 7, caractérisé en ce que le catalyseur de cuivre de Raney est en outre lavé avec de l'eau déminéralisée et de l'hydroxyde de sodium jusqu'à ce que l'eau de lavage atteigne un pH compris entre 9 et 11.

9. Procédé selon la revendication 7 ou 8, caractérisé en ce que la concentration de l'acide formique est comprise entre 20 % et 85 % en poids.
